# EUROPÄISCHE PATENTSCHRIFT

(11) **EP 0 869 817 B1**
(45) Veröffentlichungstag und Bekanntmachung des Hinweises auf die Patenterteilung: **19.06.2002**
(21) Anmeldenummer: 96943973.6
(22) Anmeldetag: 16.12.1996
(51) Int. Cl.: A61K 47/48, A61P 31/12, C07F 9/40

(54) **KOVALENTE LIPID-PHOSPHONOCARBONSÄURE-KONJUGATE UND IHRE ANWENDUNG ALS ANTIVIRALE ARZNEIMITTEL**
COVALENT LIPID-PHOSPHONO-CARBOXYLIC ACID CONJUGATES AND APPLICATION THEREOF AS ANTIVIRAL MEDICAMENTS
CONJUGUES D'ACIDES LIPIDE-PHOSPHONOCARBOXILIQUES ET LEUR APPLICATION COMME MEDICAMENTS ANTIVIRAUX

(30) Priorität: 15.12.1995 DE 19547022
(43) Veröffentlichungstag der Anmeldung: 14.10.1998
(73) Patentinhaber: Heidelberg Pharma Holding GmbH, 69126 Heidelberg (DE)
(72) Erfinder: ZILCH, Harald, D-68305 Mannheim (DE); HERRMANN, Dieter, D-69126 Heidelberg (DE); OPITZ, Hans-Georg, D-69469 Weinheim (DE); ZIMMERMANN, Gerd, D-68308 Mannheim (DE); VOSS, Edgar, D-68519 Viernheim (DE)
(74) Vertreter: Patentanwälte Zellentin & Partner
(86) Internationale Anmeldenummer: EP9605648
(87) Internationale Veröffentlichungsnummer: WO9722368

(56) Entgegenhaltungen:
- WO-A-92/03462
- WO-A-94/13682
- WO-A-97/22613
- US-A- 4 150 125
- JOURNAL OF MEDICINAL CHEMISTRY, Bd. 31, 1988, WASHINGTON US, Seiten 1831-1839, XP002036743 H. GRIENGL ET AL.: "Phosphonoformate and Phosphonoacetate Derivatives of 5-Substituted 2'Deoxyuridines: Synthesis and Antiviral Activity"
- JOURNAL OF PHARMACEUTICAL SCIENCES, Bd. 83, Nr. 9, September 1994, WASHINGTON US, Seiten 1269-1273, XP002036744 R.P.IYER ET AL.: "Synthesis, hydrolytic behavior, and anti-HIV activity of selected acyloxyalkyl esters of trisodium phosphonoformate (Foscarnet sodium)"
- PHARMAZIE, Bd. 37, Nr. 8, 1982, BERLIN DE, Seite 608 XP002036745 K.-D. THIEL ET AL: "Antivirale Aktivität von Phosphonoessigsäure, Phosphonopropionsäure und trinatriumphosphonoformiathexahydrat gegenüber Herpesvirus hominis Typ 1 und Typ 2 in vitro"

## Beschreibung

Gegenstand der vorliegenden Erfindung sind neue Lipid-Derivate von Phosphonocarbonsäuren und deren Ester der allgemeinen Formel I, 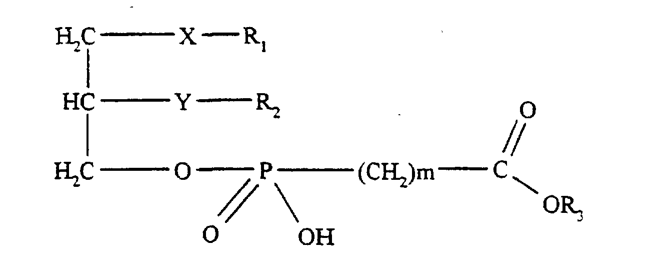 in der
- R¹: in einer Gruppierung -(CH₂)ₑ-Cycl eine geradkettige oder verzweigte, gesättigte oder ungesättigte Alkylenkette mit
- e: gleich einer ganzen Zahl zwischen 4 und 16 entspricht, wobei eines der Kohlenstoffatome ab Position 3 durch ein Heteroatom (Sauerstoff, Stickstoff oder Schwefel) ersetzt sein kann,
- R²: Wasserstoff, eine geradkettige oder verzweigte, gesättigte oder ungesättigte Alkylkette mit 1-20 Kohlenstoffatomen sein kann,
- R³: H, eine geradkettige oder verzweigte Alkykette mit 1-6 Kohlenstoffatomen, im besondern Methyl, Ethyl, Propyl, Isopropyl, Butyl, Isobutyl, t-Butyl, Pentyl, Hexyl, Neopentyl, Thexyl oder Phenyl, Cholin, Ethanolamin, Carnitin, C₅-C₇-Cycloalkylrest, Benzyl oder eine der folgenden Gruppen 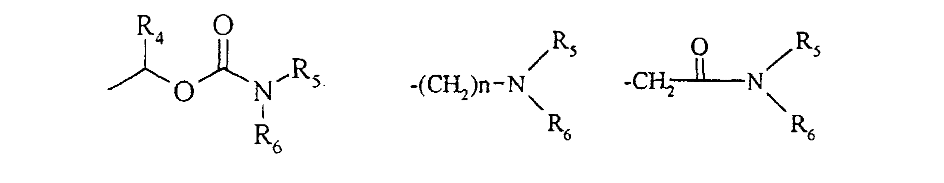 wobei R₄ C₁₋C₆-Alkyl, Benzyl oder Phenyl und R₅ und R₆ C₁₋C₆-Alkyl und n 1,2 oder
3 bedeuten kann,
- X: einen Valenzstrich, Sauerstoff, Schwefel, Oxycarbonyl, Carbonyloxy, Carbonylamido, Amidocarbonyl, die Sulfinyl- oder die Sulfonylgruppe bezeichnet,
- Y: einen Valenzstrich, Sauerstoff, Schwefel, Oxycarbonyl, Carbonyloxy, Carbonylamido, Amidocarbonyl, die Sulfinyl- oder die Sulfonylgruppe bezeichnet,
- Cycl: einen cyclischen Alkylrest mit 5-7 C-Atomen oder Phenyl darstellt, wobei ein Ring-Kohlenstoffatom durch Stickstoff ersetzt sein kann und die gesättigten oder aromatischen Ringe ein- oder mehrfach durch C₁-C₁₀-Alkyl, C₁-C₁₀-Alkoxy, C₁-C₁₀-Alkylmercapto oder Halogen substituiert sein können,
- m: für 0, 1, 2 oder 3
mit der Maßgabe, daß R₁ gleich R₂ sein kann, wenn R₂ gleichzeitig die Bedeutung von R₁ annimmt, daß heißt R₁ und R₂ in ihrer jeweiligen Bedeutung getauscht werden können deren Tautomere und deren physiologisch verträgliche Salze anorganischer und organischer Basen, sowie Verfahren zu ihrer Herstellung und diese Verbindungen enthaltende Arzneimittel.

Da die Verbindungen der allgemeinen Formel I asymmetrische Kohlenstoffatome enthalten, sind auch sämtliche optisch aktiven Formen und racemische Gemische dieser Verbindungen Gegenstand der vorliegenden Erfindung.

Die Therapie bösartiger Neoplasien (Karzinome, Sarkome, hämatologische Neoplasien), entzündlicher Erkrankungen oder Autoimmunerkrankungen sowie von durch Viren oder Retroviren hervorgerufenen Erkrankungen, wie beispielsweise von AIDS, ARC (AIDS related complex), Cytomegalie-, Herpes-Infektionen oder Hepatitis, ist neben der unzureichenden Wirksamkeit der eingesetzten therapeutischen Wirkstoffe häufig auch mit deren extremen Nebenwirkungen verbunden. Dieser Effekt ist mit der zu geringen Invivo-Selektivitat bzw. der eingeschränkten therapeutischen Breite der eingesetzten pharmakologisch aktiven Substanzen zu erklären. Die günstigen pharmakologischen Invitro-Eigenschaften der pharmakologisch aktiven Substanzen sind oft nicht auf die Invivo-Verhältnisse übertragbar.

Seit Jahren versucht man deshalb durch Modifizierung der chemischen Struktur von pharmakologisch aktiven Substanzen neue Substanzen zur Verfügung zu stellen, die verbesserte Eigenschaften hinsichtlich der therapeutischen Breite aurweisen. Ferner werden oft neue pharmazeutische Darreichungsformen mit dem Ziel entwickelt, die aktiven Substanzen gezielt an ihren Wirkungsort zu transportieren, an dem sie ihre therapeutische Wirkung entfalten sollen. Dabei soll insbesondere die unerwünschte Wechselwirkung mit gesunden Zellen vermieden werden. Im Falle von Tumorzellen, die entsprechende Oberflächenantigene besitzen, wurden beispielsweise Antikörper hergestellt, die diese speziellen Oberflächenantigene erkennen und somit gezielt an die Krebszelle binden. Die Antikörper sind mit geeigneten Toxinen derart modifziert, daß nach erfolgter Bindung an die Krebszelle das Toxin freigesetzt und die Krebszelle getötet wird. Eine andere Alternative zur Verbesserung der therapeutischen Breite besteht darin, durch geringfügige Modifizierung der pharmakologisch aktiven Substanz, beispielsweise durch Herstellung von Säure- oder Basenadditionssalzen oder durch Herstellung von pharmakologisch vertretbaren Estern [beispielsweise Fettsäureester; J. Pharm. Sci. 79, 531 (1990)], die physikalischen Eigenschaften der zugrundeliegenden aktiven Substanz derart zu verändern, daß die Löslichkeit oder Verträglichkeit der aktiven Substanz verbessert wird. Diese geringfügig chemisch modifizierten Verbindungen werden oft auch als "Prodrugs" bezeichnet, da sie beim Kontakt mit Körperflüssigkeiten oder in der Leber (first pass-Metabolismus) nahezu unmittelbar in das therapeutisch aktive Agens umgewandelt werden. Solche "Prodrugs" der Verbindungen der allgemeinen Formel I werden von dieser Erfindung mit umfaßt.

Zur Verbesserung der katabolischen Stabilität wurden Nucleoside, wie z.B. ara-C und ara-A chemisch an Phospholipide gebunden. Die entsprechenden Derivate zeigten geringere Toxizität und höhere Stabilität in vivo im Vergleich zu den unmodifizienen Nudeosiden. Absorption und Zellpenetration zeigten sich aber kaum beeinflußt [J. Med. Chem. 32, 367 (1989), Cancer Res. 37, 1640 (1977) und 41, 2707 (1981)]. Weitere Phospholipid-Derivate von Nucleosiden kennt man beispielsweise aus folgenden Literaturstellen:

In J. Biol. Chem. 265, 6112 (1990) ist die Herstellung und Verwendung von Liponucleotiden als antivirale Arzneimittel beschrieben. Untersucht und synthetisiert wurden hier aber nur die an bekannte Nucleoside, wie z.B. AZT und ddC, gekoppelten Dimyristoylphosphatidyl- und Dipalmitoylphosphatidylreste mit ihrer Fettsäureesterstruktur.

In J Med. Chem. 33, 1380(1990) sind Nucleosid-Konjugate von Thioetherlipiden mit Cytidindiphosphat beschrieben, die eine antitumorale Wirkung aufweisen und Verwendung in der Onkologie finden könnten.

In Chem Pharm. Bull. 36, 209(1988) sind 5'-(3-SN-Phosphatidyl)-nucleoside mit antileukämischer Aktivität beschrieben sowie deren enzymatische Synthese aus den entsprechenden Nucleosiden und Phosphocholinen in Gegenwart von Phospholipase D mit Transferaseaktivität.

Die enzymatische Synthese von Liponucleotiden ist u.a. ebenfalls in Tetrahedron Lett. 28, 199(1987) und Chem. Pharm. Bull. 36, 5020 (1988) beschrieben.

In WO 94/13324 sind oral verfugbare Wirkstoffe mit 1-O-Alkyl-, 1-O-Acyl-, 1-S-Acylund 1-S-Alkyl-sn-glycero-3-phosphaten als Lipid-Carrier beschrieben.

Die Anmeldung EP 418814 sowie J. Med. Chem. 34, 1912 (1991) beschreiben Isoprenoidphosphinylformiate als Squalen-Synthetase-Inhibitoren.

In Biochem. Biophys. Res. Commun. 171, 458 (1990) ist mit Palmitylphosphonoformiat ein Lipid-Konjugat des antiretroviralen Foscarnets beschrieben und in J. Med. Chem. 20, 660 (1977) wird die Anti-HIV-Aktivität von (Hexyloxy)-hydroxyphosphinylessigsäure aufgezeigt.

Es ist allgemein sehr hilfreich, effektive Wege zu finden therapeutische Arzneimittel-Konzentrationen in die entsprechenden Zielorgane bzw. Zielzellen zu transportieren, bei AIDS z.B. in die Zellen des Immunsystems und des lymphatischen Systems, die als Hauptreservoir der Virusreplikation gelten.

PFA (Phosphonoformic acid) und PAA (Phosphonoacetic acid) zeigen gute antivirale Aktivität gegen HSV 1 und 2, Influenza, HBV, VZV, EBV sowie retrovirale Infektionen.

PFA/PAA und ihre Derivate stellen unter Umständen eine effektive Alternative/Erganzung zu Nucleosiden dar, da sie ein breites Spektrum von DNA- und RNA-Polymerasen sowie die RT von Retroviren mit hinreichender Selektivität hemmen PFA und PAA selbst zeigen aufgrund ihrer Ähnlichkeit zu Pyrophosphat eine Toxizität durch Akkumulation im Knochen.

Die Verbindungen der vorliegenden Erfindung weisen ebenfalls wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papova-Viren, das Varicella-Zoster-Virus, die Hepatitis-Viren oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und 2, verursacht werden.

Besonders geeignet erscheinen die Verbindungen der Formel I zur Behandlung der klinischen Manifestationen der retroviralen HIV-Infektion beim Menschen, wie der anhaltenden generalisierten Lymphadenopathie (PGL), dem fortgeschrittenen Stadium des AIDS-verwandten Komplex (ARC) und dem klinischen Vollbild von AIDS, sowie von assoziierten CMV und HSV-Infektionen.

Für Foscarnet (Phosphonoameisensäure-trinatriumsalz/PFA) ist in J. Infect. Dis. 172, 225 (1995) der antivirale/antiretrovirale Effekt in HIV-Patienten mit CMV-Retinitis beschrieben.

Der antivirale Effekt in murinem CMV ist in Antiviral Res. 26, 1(1995) beschrieben.

Weiterhin wird in JAMA 273, 1457(1995) PFA zur Behandlung von CMV-Retinitis genutzt.

PFA- und PAA-2',3'-Didesoxy-3'-thiacytidin-Konjugate, die eine Inhibierung der HIV-1-Replikation zeigen, sind in J. Med. Chem. 37, 2216 (1994) dargestellt und in J. Pharm. Sci. 83, 1269 (1994) sind Acyloxyalkylester von Foscarnet beschrieben.

Von besonderem Interesse sind auch die US-Anmeldung 5, 194, 654 bzw. die PCT-Anmeldung WO 94/13682. Hierin sind Lipid-Derivate von Phosphonocarbonsäuren beschrieben und deren Verwendung in Liposomen unter Bildung eines besonders stabilen liposomalen Komplexes Neben einem äußerst breiten und sehr spekulativen Anspruch sind als Kern der Anmeldung 1-O-Alkyl-sn-glycero-3-phosphonocarbonsauren beschrieben, die besonders gut in die Lipiddoppelschicht von Liposomen eingebaut werden. Die beanspruchten Alkylreste können 2-24 Kohlenstoffatome umfassen, sind jedoch nicht zusätzlich substituiert.

Als Beispiel beschrieben und mit Daten für eine antivirale Wirkung belegt ist nur die Verbindung 1-O-Octadecyl-sn-glycero-3-phosphonoformiat (Batyl-Phosphonoformiat) Diese Verbindung hat sich in durchgeführten Untersuchungen und bei der Herstellung als nicht stabil herausgestellt. Im Gegensatz zu den genannten Patentanmeldungen wurde die Verbindung als Reinsubstanz in Lösung/Suspension verwendet, nicht in Liposomen.

Die erfindungsgemäßen Verbindungen der allgemeinen Formel I sind unter den gleichen Bedingungen stabil und zeigen sowohl in vitro als auch in vivo (Modell in der Maus) eindeutige Vorteile.

Die Lipidphosphonocarbonsaureester sind in vitro gleich wirksam wie die entsprechenden freien Carbonsäuren. In vivo zeigen sie hingegen eindeutige Vorteile, insbesondere bei oraler Verabreichung.
Die Carbonsaureester der Verbindungen der Formel zeigen eine im Sauren geringere Zersetzung durch Decarboxylierung und einer damit verbundene bessere Bioverfügbarkeit. Die zu verabreichende Dosis kann demnach gegenüber der entsprechenden freien Carbonsäure nochmals reduziert werden.
Weiterhin wird die Membrangängigkeit verbessert, z.B. bei der Überwindung der Blut-Hirm-Schranke und beim Passieren der Zellmembran in die Zielzelle. Da der Carbonsäureester in ivo durch Esterasen erst gespalten werden muß, wird die Halbwertszeit im Serum verlängert.

Die beanspruchten Verbindungen dieser Anmeldung stellen eine interessante Ergänzung zu WO 94/13682 und US 5, 194, 654 dar, wobei sie von diesen Anmeldungen nicht umfaßt sind.

Die Verbindungen der Formel I sind neu. Neben der besseren Stabilität (in Substanz und in Lösung) der beanspruchten Verbindungen zeigen diese auch eine bessere Wirkung im Vergleich zu den bekannten Lipid-Derivaten.

Überraschenderweise besitzen die pharmazeutischen Wirkstoffe der Formel I im Vergleich zu den pharmakologisch aktiven freien bzw unmodifizierten Substanzen eine größere therapeutische Breite. Sie verbessern darüber hinaus deren Verweilzeit im Körper, die Bioverfügbarkeit oder die oft als kritischer Faktor bekannte Membrangängigkeit (z.B. Blut-Hirn-Schranke, Zellmembran, etc.) der pharmakologisch aktiven Substanzen. Verbindungen der Formel I dienen somit als Trägersystem (Carrier) für die pharmakologisch aktiven Substanzen. Die Konjugate der Formel I können hinsichtlich ihrer Funktion als intrazelluläres Drug-Storage-, Drug-Targeting- und Drug-Delivery-System bezeichnet werden. Sie bewirken, daß die pharmakologisch aktive Substanz nach oraler Applikation intrazellulär freigesetzt wird, wobei diese Freisetzung in vorteilhafter Weise nicht unspezifisch in allen Zellen, Organen oder Geweben des Körpers stattfindet, sondern gezielt in solchen Zellen, die ein bestimmtes Enzym enthalten Besonders überraschend ist jedoch, daß die Spaltung nicht bereits schon während des Transportes des Substrates durch die Körperflüssigkeiten, wie z.B. Blut, Serum oder Lymphflüssigkeit oder durch die Leber, erfolgt, sondern erst an oder in den entsprechenden Zielzellen. Auf diese Weise wird das unerwünschte Ausscheiden der Phosphonocarbonsäure durch die Niere oder Spaltung des Konjugats in der Leber vermieden, so daß der weitaus größte Teil des Wirkstoffes an bzw. in die jeweiligen Zielzellen transportiert wird. Derartige Zellen sind, wie oben bereits erwähnt, insbesondere physiologisch oder pathophysiologisch aktivierte Zellen, die als Zielobjekt für die Verabreichung von pharmakologisch aktiven Substanzen in Frage kommen, wie beispielsweise Blutleukozyten, Lymphozyten, Makrophagen und andere Zellpopulationen des immunologisch lymphatischen Systems. Insbesondere handelt es sich hierbei um aktivierte Zellen (z.B. Makrophagen, Granulozyten, Lymphozyten, Leukozyten, Thrombozyten, Monozyten etc.), die beim jeweiligen Krankheitsgeschehen eine pathophysiologische oder symptomatische Rolle spielen. Darüber hinaus handelt es sich auch um Zellen, die durch Viren, Bakterien, Pilze oder andere Mikroorganismen infiziert sind.

Überraschenderweise wurde auch gefunden, daß die therapeutische Breite einer pharmakologisch aktiven Phosphonocarbonsäure und deren Ester signifikant verbessert wird, wenn die Substanz an ein sehr spezielles lipidartiges Trägermolekül gekoppelt wird. Das so hergestellte Konjugat dient als neuer Wirkstoff für die Herstellung von pharmazeutischen Darreichungsformen. Insgesamt resultiert aus der Kopplung eine verstärkte Wirkung der pharmazeutisch aktiven Phosphonocarbonsäure in vivo, da durch das entstehende Drug-Delivery-Transport-System eine Lokalisierung der pharmakologische aktiven Substanz in Zielzellen erfolgt und dadurch die pharmakologisch aktive Substanz hinsichtlich ihrer Effizienz und Verträglichkeit verbessert wird. Dies bedeutet, daß einerseits die Menge der zu verabreichenden pharmakologisch aktiven Phosphonocarbonsäure reduziert werden kann oder andererseits unter Beibehaltung der gleichen effektiven Menge eine verstärkte pharmakologische Wirkung erzielt wird.

Aus dem Konjugat wird die pharmakologisch aktive Phosphonocarbonsäure durch enzymatische Hydrolyse des Konjugats freigesetzt.

Die Konjugate der Formel I zeigen entscheidende Vorteile im Vergleich zur nicht konjugierten pharmakologisch aktiven Phosphonocarbonsäure bzw. deren Ester. Der spezifische, kovalent an die pharmakologisch aktive Substanz gebundene Carrier verbessert die Bioverfügbarkeit der schlecht resorbierten pharmakologisch aktiven Substanzen, die Verträglichkeit der potentiell toxischen Wirkmolekülen, die Verweilzeit der schnell eliminierten oder metabolisierten Arzneimitteln und die Membranpenetration der schlecht membrangängigen Verbindungen (z.B. Blut-Hirn, Zellen etc ).

Die enzymatische Spaltung des Lipidteils in vivo erfolgt in der Regel nicht im Serum sondern erst intrazellulär Außerdem verbessert der Carrierteil mit seiner lecithinartigen Struktur, die für den beanspruchten Effekt essentiell ist, die Penetration oder Membrangangigkeit der pharmakologisch aktiven Substanz und zeigt einen Depoteffekt Darüber hinaus ist die gastrointestinale Verträglichkeit der Lipidkonjugate vielfach besser als die der reinen pharmakologisch aktiven Phosphonocarbonsäure. Auch bei der Resorption zeigt das Lipid-Konjugat eine bessere Penetration durch Membranstrukturen und somit eine bessere Überwindung der Resorptionsbarrieren. Entsprechendes gilt für die Penetration z.B. der Blut-Hirn-Schranke.

Durch eine bessere Bindung des Konjugats an Plasma- und Gewebeproteine wird ferner die Verteilung in vivo verbessert. Durch normale Biotransformation wird das Konjugat primär vom Thioether zum Sulfoxid oxidiert, was aber aufgrund der equipotenten Wirkung des Sulfoxids im Vergleich zum Thioether keinen Nachteil darstellt Durch eine langsame Freisetzung der pharmakologisch aktiven Phosphonocarbonsäure aus dem Konjugat wird ein niedriger, aber uber einen längeren Zeitraum konstanter aktiver Substanzspiegel gewährleistet und somit die Wirkung verbessert und/oder toxische Nebeneffekte vermieden. Die freigesetzte pharmakologisch aktive Substanz in Form des Monophosphats penetriert wegen seiner großen Hydrophilie nicht mehr aus der Zelle heraus.

Sowohl die Gesamtkörper-, Zell- als auch die Organ-Halbwertszeiten der pharmakologisch aktiven Substanz werden durch Konjugation aufgrund der längeren Verweilzeit des Konjugats im Organismus erheblich verlängert. Aufgrund der fehlenden Spaltungsaktivität im Serum und in verschiedenen Organen ist nahezu keine bzw nur eine sehr geringere Knochenmark- und Organtoxizitat zu beobachten. Insbesondere ist vorteilhaft, daß die Konjugate der Formel I in verschiedenen Zielorganen, Geweben oder Zellen spezifisch angereichert werden.

Die Verbindung der Formel I können als Wirkstoffe zur Herstellung von Arzneimitteln verwendet werden, die für alle Erkrankungen eingesetzt werden, bei denen hohe pharmakologisch aktive Substanzspiegel in Zellen, Organen oder Geweben erforderlich oder hilfreich sind. Eine wesentliche Voraussetzung für dieses als "Drug-Storage-Delivery-Targeting" zu bezeichnende System ist, daß die im Sinne der beabsichtigten Therapie anzusprechenden Zellen das Spaltungsenzym haben, so daß in einem ersten Schritt der Wirkstoff bindet und anschließend über die Zellmembran hinweg in das Innere der Zelle transportiert wird, wobei die Spaltung des Wirkstoffes zu der physiologisch aktiven Phosphonocarbonsäure entweder im wesentlichen gleichzeitig mit dem Transport über die Zellmembran oder auch später teilweise innerhalb der Zelle erfolgt. Die intrazelluläre Spaltung kommt insbesondere in solchen Fällen vor, bei denen das Spaltungsenzym auch innerhalb der Zelle lokalisiert ist.

Geeignete Zielzellen sind beispielsweise Zellen des immunologisch-lymphatischen Systems (z.B Blutleukozyten, Monozyten, Makrophagen, Lymphozyten) oder infizierte Zellen.

Überraschenderweise wurde auch gefunden, daß Verbindungen der allgemeinen Formel I die Vermehrung von DNA- bzw. RNA-Viren auf der Stufe der virusspezifischen DNAbzw RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw Nature 325, 773, 1987). Von besonderem therapeutischen Interesse ist die Hemmwirkung auf das HI-Virus, dem Verursacher der Immunschwäche-Erkrankung AIDS. Zur Behandlung von AIDS ist heute u.a. 3'-Azido-3 desoxythymidin (DE-A-3608606) bei AIDS Patienten zugelassen. Jedoch machen toxische Nebenwirkungen des 3'-Azido-3'-desoxythymidins auf das Knochenmark bei etwa 50 % der behandelten Patienten Bluttransfusionen erforderlich. Die Verbindungen der allgemeinen Formel I besitzen diese Nachteile nicht. Sie wirken antiviral, ohne in pharmakologisch relevanten Dosen zyloloxisch zu sein.

Die Verbindungen der vorliegenden Erfindung und ihre pharmazeutischen Zubereitungen können auch in Kombination mit anderen Arzneimitteln zur Behandlung und Prophylaxe der oben genannten Infektionen eingesetzt werden. Beispiele dieser weiteren Arzneimittel beinhalten Mittel, die zur Behandlung und Prophylaxe von HIV-Infektionen oder diese Krankheit begleitende Erkrankungen einsetzbar sind wie 3'-Azido-3'desoxythymidin, 2',3'-Didesoxynucleoside wie z.B. 2',3'-Didesoxycytidin, 2',3'-Didesoxyadenosin und 2',3'-Didesoxyinosin, acyclische Nucleoside (z.B Acyclovir), nicht-nukleosidische RT-Inhibitoren, Proteaseinhibitoren wie z B. Invirase, Interferone wie z.B. Interferon α, β, γ, Cytokine und Interleukine (z.B. Interleukin 16), Chemokine wie z.B. MIP1α, MIP1β, CC1, renale Ausscheidungs-lnhibitoren wie z.B. Probenicid, Nucleosid-Transport-Inhibitoren wie z.B. Dipyridamol, als auch Immunmodulatoren wie z.B. Interleukin II oder Stimulierungs-Faktoren wie z.B. Granulozyten-Makrophagen-Kolonie stimulierende Faktoren (GM-CSF), Granulozyten-Kolonie stimulierende Faktoren (G-CSF, Neutropoetin), Thrombopoetin und thrombopoetin-ähnliche Faktoren Die Verbindungen der vorliegenden Erfindung und das andere Arzneimittel können jeweils einzeln, gleichzeitig gegebenenfalls in einer einzigen oder zwei getrennten Formulierungen oder zu unterschiedlichen Zeiten verabreicht werden, so daß ein synergistischer Effekt erreicht wird.

Als mögliche Salze der Verbindungen der allgemeinen Formel I kommen vor allem Alkali-, Erdalkali- und Ammoniumsalze der Carboxyl- und Phosphonatgruppe in Frage. Als Alkalisalze sind Lithium-, Natrium- und Kaliumsalze bevorzugt Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1-4 Kohlenstoffatomen und/oder Aralkylreste, bevorzugt Benzylreste, substituiert sein kann. Die Substituenten können hierbei gleich oder verschieden sein.

In der Formel I bedeutet R¹ vorzugsweise eine geradkettige, gesättigte Alkylenkette mit e gleich 5-12 Kohlenstoffatomen. Cycl stellt vorzugsweise den Cyclohexyl- oder Cyclopentylrest bzw. Phenyl dar, das gegebenenfalls durch C₁-C₄-Alkyl oder Halogen substituiert ist. X und Y sind unabhängig voneinander bevorzugt gleich Schwefel, Sulfinyl, Sulfonyl, Sauerstoff oder Valenzstrich. Besonders bevorzugt ist X gleich Schwefel und Y gleich Sauerstoff. Der Rest -(CH₂)ₑ-Cycl steht bevorzugt in der 3-Position des C₃-Grundkörpers. e bedeutet besonders bevorzugt einen Bereich von 6-10 (CH₂)ₑ-Cycl bedeutet ganz besonders bevorzugt Phenylhexyl oder Cyclohexl-hexyl. Bevorzugte Alkylreste für R² sin geradkettige oder verzweigte, gesättigte oder ungesättigte ASkylketten mit 8-12 Kohlenstoffatomen. Besonders bevorzugte Alkylreste für R² sind die Nonyl-, Decyl-, Undecyl- oder Dodecylgruppe.

Besonders bevorzugte, gekoppelte Phosphonocarbonsäuren in den beanspruchten Konjugaten der allgemeinen Formel I sind:
- Phosphonoameisensäure
- Phosphonoessigsäure
- Phosphonopropionsäure

Bevorzugte Ester der Phosphonoameisen-, Phosphonoessig- und Phosphonopropionsäure sind der Methylester, Ethylester und Propylester, Butylester, t-Butylester und Benzylester
Die Verbindungen der allgemeinen Formel I können dargestellt werden, in dem man
1. eine Verbindung der allgemeinen Formel II, 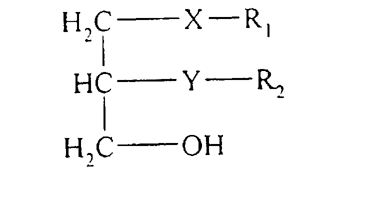 in der R¹, R² und n die angegebenen Bedeutungen besitzen, mit einer Verbindung der allgemeinen Formel III, 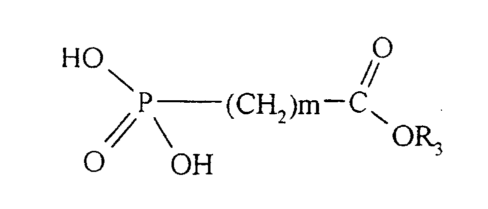 in der m und R³ die oben angegebene Bedeutung besitzt und R³ bevorzugt einen C₁-C₆-Alkylrest darstellt, in Gegenwart von einem gegebenenfalls substituierten Arylsulfonsäurechlorid in einer organischen Base, bzw. in Gegenwart der Base in einem inerten organischen Lösungsmittel umsetzt und anschließend den Carbonsäureester durch alkalische Verseifung in ein Derivat der Formel I bzw. dessen physiologisch verträgliches Salz überführt;
   oder
2. ein gemischtes Anhydrid aus einer Verbindung der Formel III und einem Alkyloder Arylsulfonsäurechlorid herstellt und in Gegenwart einer Base in einem inerten organischen Lösungsmittel bzw. direkt in der Base mit einem Alkohol der Formel II zur Reaktion bringt und anschließend den Carbonsäureester gegebenenfalls alkalisch verseift;
   oder
3. eine Phosphonocarbonsäure der Formel III, in der R³ gleich Wasserstoff ist, mit einem Alkohol der Formel II in Gegenwart einer Base und eines gegebenenfalls substituierten Arylsulfonsäurechlorids umsetzt und nach Bedarf in ein physiologisch verträgliches Salz oder Ester überführt;
   oder
4. ein gemischtes Anhydrid aus einer Verbindung der Formel III, in der R³ gleich Wasserstoff ist, und einem Alkyl- oder Arylsulfonsäurechlorid in Gegenwart einer Base, evtl. in einem inerten organischen Lösungsmittel, mit einem Alkohol der Formel II zur Reaktion bringt und das Konjugat gegebenenfalls in ein physiologisch verträgliches Salz überführt.
   oder
5. Phosphonsäuredichloride, der allgemeinen Formel IV, 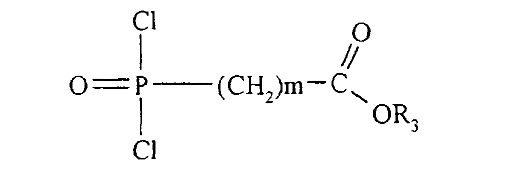 die sich nach Bhongle et al. (Synthetic Commun. **17**, 1071 (1987)) ausgehend von einem Phosphonsäure-bis-trimethylsilylester durch anschließende Umsetzung mit Oxalylchlorid darstellen lassen, mit einem Alkohol der allgemeinen Formel 11 in Gegenwart einer Base im Molverhältnis 1 : 1 umgesetzt
   oder
6 eine Verbindung der Formel III mit Oxalylchlorid, wie in Tetrahedron Letters **33,** 7473(1992) beschrieben, in das entsprechende Phosphonsäuredichlorid der Formel IV überführt, das anschließend mit einem Alkohol der Formel II in Gegenwart einer Base im Molverhältnis 1:1 umgesetzt wird. Das als Zwischenprodukt entstehende Phosphonsäure-mono-chlorid wird zum Halbester verseift und der Carbonsäureester durch alkalische Verseifung in ein Derivat der Formel I bzw. dessen physiologisch verträgliches Salz überführt.

Die Herstellung von Verbindungen der allgemeinen Formel II sind exemplarisch in den Beispielen in EP 0545966 beschrieben.

Die Arzneimittel enthaltenden Verbindungen der Formel I zur Behandlung von z.B. viralen Infektionen können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nicht toxischen Salze, hochmolekulare Polymere, wie flüssiges Polyethylenoxid zur Viskositätsregulierung.

Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdispere Kieselsauren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- und Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden Die Tabletten können auch retardiert sein, wodurch sich die Anzahl der Applikationen pro Tag auf 1-3 vermindert Der Wirkstoffgehalt der retardierten Tabletten kann 2-1000 mg betragen. Der Wirkstoff kann auch durch Dauerinfusion gegeben werden, wobei die Mengen von 5-5000 mg pro Tag normalerweise ausreichen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage :
1. ((3-(4-Chlorphenyl)hexylmercapto)-2-decyloxy)-propoxy)-hydroxyphosphinylameisensäure
2. ((3-(6-Cyclohexylhexylmercapto)-2-decyloxy)-propoxy)-hydroxyphosphinylameisensäureisopropylester
3. [3-(Phenyl)oxy-hexylmercapto-2-decyloxy]propoxy-hydroxyphosphinylameisensäure
4. [3-(Phenyl)heptylmercapto-2-decyloxy]propoxy-hydroxyphosphinylameisensäure
5. ((3-(6-Cyclohexylhexylmercapto)-2-decyloxy)-propoxy)-hydroxyphosphinylameisensäurepentylester
6. [3-(m-Ethylphenyl)decylmercapto-2-decyloxy]propoxy-hydroxyphosphinylameisensäure
7. [3-(p-tert-Butylphenyl)octylmercapto-2-decyloxy]propoxy-hydroxyphosphinylameisensäure
8. [3-(Cyclohexyl )heptylmercapto-2-decyloxy]propoxy-hydroxyphosphinylameisensäure
9. [3-(Cyclopentyl)nonylmercapto-2-decyloxy]propoxy-hydroxyphosphinylameisensäure
10. [3-(Cycloheptyl)octylmercapto-2-decyloxy]propoxy-hydroxyphosphinylameisensäure
11. [3-(Cyclohexyl)oxy-pentylmercapto-2-decyloxy]propoxy-hydroxyphosphinylameisensäure
12. [3-(Cyclohexyl)mercapto-pentylmercapto-2-decyloxy]propoxyhydroxyphosphinyl-ameisensäure
13. [3-(Phenyl)undecylmercapto-2-decyloxy]propoxy-hydroxyphosphinylameisensäure
14. [3-Dodecylmercapto-2-(phenyl)hexylmercapto]propoxy-hydroxyphosphinyl ameisensäure
15. [3-Decyloxy-2-(cyclohexyl)-hexylmercapto]-propoxy hydroxyphosphinylameisensäure
16. [3-(p-Chlorphenyl) hexylmercapto-2-decyloxy] propoxy-hydroxyphosphinylessigsäure
17. [3-(p-tert-Butylphenyl) oxy-octylmercapto-2-decyloxy] propoxy-hydroxyphosphinyl-essigsäure
18. ((3-(6-Cyclohexylhexylmercapto)-2-decyloxy)-propoxy)-hydroxyphosphinylameisensäurebenzylester
19. [3-(Phenyl) heptylmercapto-2-decyloxy] propoxy-hydroxyphosphinyl-essigsäure
20. [3-(p-Chlorphenyl) oxy-pentylmercapto-2-decyloxy] propoxyhydroxyphosphinyl-essigsäure
21. [3-(m-Ethylphenyl) decylmercapto-2-decyloxy] propoxy-hydroxyphosphinyl-essigsäure
22. [3-(p-tert-Butylphenyl) octylmercapto-2-decyloxy] propoxy-hydroxyphosphinyl-essigsäure
23. [3-(Cyclohexyl) heptylmercapto-2-decyloxy] propoxy-hydroxyphosphinyl-essigsäure
24. [3-(Cyclopentyl) nonylmercapto-2-decyloxy] propoxy-hydroxyphosphinyl-essigsäure
25. [3-(Cycloheptyl) octylmercapto-2-decyloxy] propoxy-hydroxyphosphinyl-essigsäure
26. [3-(Cyclohexyl) oxy-pentylmercapto-2-decyloxy] propoxy-hydroxyphosphinyl-essigsäure
27. [3-(Cyclohexyl) mercapto-pentylmercapto-2-decyloxy] propoxy-hydroxyphosphinyl-essigsäure
28. [3-(Phenyl) undecylmercapto-2-decyloxy] propoxy-hydroxyphosphinyl-essigsäure 29 [3-Dodecylmercapto-2-(phenyl) hexylmercapto] propoxy hydroxyphosphinyl essigsäure
30. [3-Decyloxy-2-(cyclohexyl) hexylmercapto] propoxy hydroxyphosphinyl essigsäure
31. [3-(p-Chlorphenyl) hexylmercapto-2-decyloxy] propoxy-hydroxyphosphinylpropion-säure
32. [3-(p-tert-Butylphenyl) oxy-octylmercapto-2-decyloxy] propoxyhydroxyphosphinyl-propionsäure
33. [3-(Phenyl) oxy-hexylmercapto-2-decyloxy] propoxy-hydroxyphosphinylpropionsäure
34. [3-(Phenyl) heptylmercapto-2-decyloxy] propoxy-hydroxyphosphinylpropionsäure
35. [3-(p-Chlorphenyl) oxy-pentylmercapto-2-decyloxy] propoxyhydroxyphosphinyl-propionsäure
36. [3-(m-Ethylphenyl) decylmercapto-2-decyloxy] propoxy-hydroxyphosphinylpropionsäure
37. [3-(p-tert-Butylphenyl)octylmercapto-2-decyloxy]propoxy-hydroxyphosphinylpropionsäure
38. [3-(Cyclohexyl) heptylmercapto-2-decyloxy]propoxy-hydroxyphosphinylpropionsäure
39. [3-(Cyclopentyl) nonylmercapto-2-decyloxy] propoxy-hydroxyphosphinylpropionsäure
40. [3-(Cycloheptyl) octylmercapto-2-decyloxy] propoxy-hydroxyphosphinylpropionsäure
41. [3-(Cyclohexyl) oxy-pentylmercapto-2-decyloxy] propoxy-hydroxyphosphinylpropionsäure
42. [3-(Cyclohexyl) mercapto-pentylmercapto-2-decyloxy] propoxyhydroxyphosphinyl-propionsäure
43. [3-(Phenyl) undecylmercapto-2-decyloxy] propoxy-hydroxyphosphinylpropionsäure
44. [3-Dodecylmercapto-2-(phenyl) hexylmercapto] propoxy phospinyl propionsäure
45. [3-Decyloxy-2-(cyclohexyl) hexylmercapto] propoxy hydroxyphosphinyl propionsäure
46. ((3-(6-Cyclohexylhexylmercapto)-2-decyloxy)-propoxy)-hydroxyphosphinylameisensäureisobutylester
47. ((3-(6-Phenylhexylmercapto)-2-decyloxy)-propoxy)-hydroxyphosphinylameisensäureethylester
48. ((3-(6-Phenylhexylmercapto)-2-decyloxy)-propoxy)-hydroxyphosphinylameisensäurepropylester
49. ((3-(6-Phenylhexylmercapto)-2-decyloxy)-propoxy)-hydroxyphosphinylameisensäure-t-butylester
50. ((3-(6-Phenylhexylmercapto)-2-decyloxy)-propoxy)-hydroxyphosphinylameisensäure-(2-dimethylamino)ethylester

### Beispiel 1

### R,S-((3-(6-Phenylhexylmercapto)-2-decyloxy)-propoxy)-hydroxyphosphinylameisensäure Dinatriumsalz 1 (Ph6S10OP-PFA)

### 6-Phenyl-1-hexanthiol 13

Unter Stickstoff gab man 15.0 g (62.2 mmol) 1-Brom-6-phenyl-hexan (beschrieben in der Offenlegungsschrift der int. Anm. PCT/EP95/04413) gelöst in 40 ml Ethanol zu einer Lösung aus 7.10 g (93.3 mmol) Thioharnstoff in 30 ml Ethanol. Nach 7 h bei Rückflußtemp., ließ man auf Raumtemp. abkühlen, versetzte mit 33 ml konz. Ammoniak und erhitzte 4 h zum Rückfluß. Anschließend wurde mit 15 ml conc. HCI auf pH 1 angesäuert. Man extrahierte dreimal mit je 200 ml Ether, wusch mit Wasser und gesättigter Natriumchloridlsg., trocknete über Magnesiumsulfat und entfernte das Lösungsm. i. Vak. Der Rückstand wurde in Dichlormethan aufgenommen, der Feststoff abgesaugt, mit Dichlormethan nachgewaschen und das Filtrat i. Vak. eingedampft. 9.80 g (82%) **13** als farbloses Öl.

### R,S-2-Decyloxy-3-(6-phenylhexylmercapto)-1-propyl-benzoat 14

Unter Stickstoffatmosphäre legte man 9.60 g (49.4 mmol) **13** und 6.80 g (49.4 mmol) Kaliumcarbonat in 100 ml Methylethylketon vor, rührte 15 Min. und versetzte dann mit 19.7 g (49.4 mmol) 3-Brom-2-decyloxy-1-propyl-benzoat **12** (EP 0545966) und einem Kristall Kaliumiodid. Nach Zugabe von 5 ml Dimethylformamid wurde 48 h bei Raumtemp. gerührt. Man saugte vom Kaliumcarbonat ab, wusch den Niederschlag mit Heptan und engte das Filtrat i. Vak. ein. Der Rückstand wurde in Wasser aufgenommen, mit Heptan extrahiert und die organische Phase mit 0.5 N NaOH gewaschen, mit Wasser neutralisiert, über Magnesiumsulfat getrocknet und eingedampft. Man erhielt 25.6 (100%) **14,** welches ohne weitere Reinigung zur Synthese von **15** eingesetzt wurde.

### R,S-2-Decyloxy-3-(6-phenylhexylmercapto)-1-propanol 15

Unter Stickstoff rührte man eine Mischung aus 25.5 g (49.7 mmol) **14,** 30 ml Ethanol und 12 ml (60.0 mmol) 5 N NaOH insgesamt 48 h bei Raumtemp. Man dampfte i. Vak. ein, nahm in Wasser auf, extrahierte mit Dichlormethan, wusch mit 1 N NaOH, Wasser, trocknete über Magnesiumsulfat und entfernte das Lösungsm. i. Vak. Es wurden 18.9 g (93%) Rohprodukt erhalten. Reinigung erfolgte durch Flashchromatographie an Kieselgel (Laufmittel: Heptan/Essigester 7:1), wobei 12.8 g (63%) **15** als farbloses Öl isoliert wurden.

### Dichlorphosphinylameisensäuremethylester 16

Unter Stickstoff wurden 28.2 g (99.2 mmol) Di-(trimethylsilyloxy)-hydroxyphosphinylameisensäuremethylester (Synthetic Commun. **17**, 1071(1987); Tetrahedron Lett. **33**, 7473) in 150 ml Dichlormethan und 5 Tropfen Dimethylformamid gelöst und bei 0°C innerhalb von 30 Min 37.8 g (0.297 mol) Oxalylchlorid zugetropft. Nach 2 h Rühren bei Raumtemp. entfernte man Lösungsm. i. Vak. und destillierte im Hochvakuum. 12.1 g (69%) **16**, Sdp. 42-45°C/0.19 mbar.

### R,S-((3-(6-Phenylhexylmercapto)-2-decyloxy)-propoxy)-hydroxyphosphinylameisensäuremethylester 17 (Beispiel 12.21)

Unter Stickstoffatmosphäre wurden 1.50 g (8.48 mmol) Dichlorphosphinylameisensäuremethylester **16** in 15 ml Dichlormethan gelöst und auf 5°C abgekühlt. Man tropfte innerhalb von 15 Min. eine Mischung aus 3.50 g (8.48 mmol) R,S-2-Decyloxy-3-(6-phenylhexylmercapto)-1-propanol **15** und 900 mg (8.48 mmol) Triethylamin gelöst in 20 ml Dichlormethan zu, wobei die Innentemperatur auf 10°C anstieg. Nach 30 Min. bei 10° C wurde noch 3 h bei Raumtemp. gerührt und anschließend in eine Lösung aus 7.85 ml 1 N NaOH und 200 ml Eiswasser gegossen. Man extrahierte zweimal mit je 100 ml Dichlormethan, wusch die kombinierten organischen Phasen mit Wasser und trocknete über Magnesiumsulfat. Nach Entfernung des Lösungsm. i. Vak. erhielt man 4.3 g (95%) eines Öls, welches durch Flashchromatographie an Kieselgel gereinigt wurde. Nach Elution von unverbrauchtem **15** (1.35 g, Laufmittel: Essigester) ergab die Entwicklung mit Dichlormethar/Methanol 10:1 insgesamt 2.52 g (56%) **17** (Beispiel 21) als farbloses Öl.

Unter Stickstoff versetzte man eine Mischung aus 2.50 g (4.71 mmol) **17**, 20ml Ethanol und 20 ml Tetrahydrofuran mit 4.7 ml (14.1 mmol) 3 N NaOH. Es wurde 2 h bei Raumtemp. gerührt, das Lösungsm. am Rotationsverdampfer abgezogen, in 250 ml Wasser aufgenommen und zweimal mit je 50 ml t-Butylmethylether extrahiert. Die wässrige Phase brachte man mit 1 N HCl auf pH 8.5 und entfernte das Wasser durch Gefriertrocknung. 2.3 g (87%) **1,** Schmp. 212-214°C.

### Beispiel 2

### R,S-((3-(12-Phenyldodecylmercapto)-2-decyloxy)-propoxy)-hydroxyphosphinylameisensäure Dinatriumsalz 2 (Ph12S10OP-PFA)

### 12-Phenyl-1-dodecanthiol 18

Wie bei der Darstellung von **13** (Beispiel 1) wurden 15.0 g (46.1 mmol) 1-Brom-12-phenyl-dodecan mit 5.3 g (69.2 mmol) Thioharnstoff umgesetzt, 11.1 g (87%) **18.**

### R,S-Decyloxy-3-(12-phenyldodecylthio)-propyl-benzoat 19

10.8 g (38.8 mmol) **18** und 15.3 g (38.8 mmol) **12** ergaben 20.0 g (92%) **19.**

### R,S-Decyloxy-3-(12-phenyLdodecylthio)-1-propanol 20

Die Hydrolyse von 4.40 g (7.37 mmol) **19** mit 3.0 ml (15 mmol) 5 N NaOH lieferte 3.08 g (85%) **20** als farbloses Öl.

Analog zu Beispiel 1 wurden aus 1.90 g (9.95 mmol) **16** und 4.90 g (9.95 mmol) R,S-2-Decyloxy-3-(12-phenyldodecylmercapto)-1-propanol **20** 3.39 g (52%) R,S-((3-(12-Phenyl-dodecylmercapto)-2-decyloxy)-propoxy)-hydroxyphosphinylameisensäuremethylester (Beispiel 12.22) als farbloses Öl erhalten. Verseifung mit Natronlauge (analog Beispiel 1) ergab 2.90 g (94%) **2** vom Schmp. 224°C.

### Beispiel 3

### R,S-((3-(10-Phenyldecylmercapto)-2-decyloxy)-propoxy)-hydroxvphosphinylameisensäure Dinatriumsalz 3 (Ph10S10OP-PFA)

Analog zu Beispiel 1 wurden aus 1.10 g (6.29 mmol) **16** und 2.92 g (6.29 mmol) R,S-2-Decyloxy-3-(10-phenyldecylmercapto)-1-propanol 0.85 g (23%) R,S-((3-(10-Phenyldecylmercapto)-2-decyloxy)-propoxy)-hydroxyphosphinyl-ameisensäuremethylester (Beispiel 12.23) als farbloses Harz erhalten. Verseifung mit Natronlauge (analog Beispiel 1) ergab 0.71 g (79%) **3** vom Schmp. 219-220°C.

### Beispiel 4

### R,S-((3-(5-(4-Chlorphenyl)-pentylmercapto)-2-decyloxy)-propoxy)-hydroxyphosphinylameisensäure Dinatriumsalz 4 (ClPh5S10OP-PFA)

Analog zu Beispiel 1 wurden aus 1.10g ( mmol) **16** und 2.70 g (6.20 mmol) R,S-2-Decyloxy-3-(5-(4-chlorphenyl)-pentylmercapto)-1-propanol 3.30 g (97%) R,S-((3-(5-(4-chlorphenyl)-pentylmercapto)-2-decyloxy)-propoxy)-hydroxyphosphinylameisensäuremethylester (Beispiel 12.24) als farbloses Öl erhalten. Verseifung von 2.80 g des Esters mit Natronlauge (analog Beispiel 1) ergab 2.90 g (96%) **4** vom Schmp. 170-172°C.

### Beispiel 5

### R,S-((3-(10-(4-t-Butylphenoxy)-decylmercapto)-2-decyloxy)-propoxy)-hydroxyphosphinyl-ameisensäure Dinatriumsalz 5 (tBuPhO10S10OP-PFA)

Analog zu Beispiel 1 wurden aus 1.10g ( 6.20 mmol) **16** und 3.34 g (6.20 mmol) R,S-2-Decyloxy-3-(5-(4-t-butylphenoxy)-decylmercapto)-1-propanol 1.92 g (58%) R,S-((3-(5-(4-t-Butylphenoxy)-decylmercapto)-2-decyloxy)-propoxy)-hydroxyphosphinylameisensäuremethylester (Beispiel 12.25) als farbloses Öl erhalten. Verseifung mit Natronlauge (analog Beispiel 1) ergab 1.90 g (95%) **5.**

### Beispiel 6

### R,S-((3-(5-Cyclohexylpentylmercapto)-2-decyloxy)-propoxy)-hydroxyphosphinylameisensäure Dinatriumsalz 6 (CH5S10OP-PFA)

Analog zu Beispiel 1 wurden aus 1.10 g (6.20 mmol) **16** und 2.48 g (6.20 mmol) R,S-2-Decyloxy-3-(5-cyclohexylpentylmercapto)-1-propanol 2.60 g (81%) R,S-((3-(5-cyclohexylpentylmercapto)-2-decyloxy)-propoxy)-hydroxyphosphinylameisensäuremethylester (Beispiel 12.26) als farbloses Öl erhalten. Verseifung mit Natronlauge (analog Beispiel 1) ergab 1.50 g (92%) **6** vom Schmp. 217-219°C.

### Beispiel 7

### R,S-((3-(6-Cyclohexylhexylmercapto)-2-decyloxy)-propoxy)-hydroxyphosphinylameisensäure Dinatriumsalz 7 (CH6S10OP-PFA)

Analog zu Beispiel 1 wurden aus 1.30 g (7.30 mmol) **16** und 3.00 g (7.30 mmol) R,S-2-Decyloxy-3-(6-cyclohexylhexylmercapto)-1-propanol 2.80 g (72%) R,S-((3-(6-cyclohexylhexylmercapto)-2-decyloxy)-propoxy)-hydroxyphosphinyl-ameisensäuremethylester (Beispiel 12.27) als farbloses Öl erhalten. Verseifung von 2.02 g dieses Esters mit Natronlauge (analog Beispiel 1) ergab 2.00 g (93%) **7** vom Schmp. 199-202°C.

### Beispiel 8

### R,S-((3-(12-Cyclohexyldodecylmercapto)-2-decyloxy)-propoxy)-hydroxyphosphinylameisensäure Dinatriumsalz 8 (CH12S10OP-PFA)

Analog zu Beispiel 1 wurden aus 0 55 g (3.10 mmol) **16** und 1.50 g (3.10 mmol) R,S-2-Decyloxy-3-( 12-cyclohexyldodecylmercapto)-1-propanol 1.70 g (81%) R,S-((3-(12-cyclohexyldodecylmercapto)-2-decyloxy)-propoxy)-hydroxyphosphinyl-ameisensäuremethylester (Beispiel 12.28) als farbloses Öl erhalten. Verseifung von 1.50 g dieses Esters mit Natronlauge (analog Beispiel 1) ergab 1.10 g (71%) **8** vom Schmp. 105-107°C.

### Beispiel 9

### R,S-((3-(8-Cyclohexyloctylmercapto)-2-decyloxy)-propoxy)-hydroxyphosphinylameisensäure Dinatriumsalz 9 (CH8S10OP-PFA)

Analog zu Beispiel 1 wurden aus 1.10 g (6.29 mmol) **16** und 2.75 g (6.29 mmol) R,S-2-Decyloxy-3-(8-cyclohexyloctylmercapto)-1-propanol 2.40 g (68%) R,S-((3-(8-cyclohexyloctylmercapto)-2-decyloxy)-propoxy)-hydroxyphosphinyl-ameisensäuremethylester (Beispiel 12.29) als farbloses Öl erhalten. Verseifung von 1.37 g des erhaltenen Esters mit Natronlauge (analog Beispiel 1) ergab 0.95 g (68%) **9**, Zers. > 250°C.

### Beispiel 10

### R,S-((3-(10-Cyclohexyldecylmercapto)-2-decyloxy)-propoxy)-hydroxyphosphinylameisensäure Dinatriumsalz 10 (CH10S10OP-PFA)

Analog zu Beispiel 1 wurden aus 1.10 g (6.29 mmol) **16** und 2.96 g (6.29 mmol) R,S-2-Decyloxy-3-(10-cyclohexyldecylmercapto)-1-propanol 1.15 g (37%) R,S-((3-(10-cyclohexyldecylmercapto)-2-decyloxy)-propoxy)-hydroxyphosphinyl-ameisensäuremethylester (Beispiel 12.30) als farbloses Öl erhalten. Verseifung mit Natronlauge (analog Beispiel 1) ergab 1.06 g (89%) **10** vom Schmp. 179-181°C

### Beispiel 11

### R,S-((3-(5-(4-Chlorphenoxy)-pentylmercapto)-2-decyloxy)-propoxy)-hydroxyphosphinyl-ameisensäure Dinatriumsalz 11 (ClPhO5S10OP-PFA)

Analog zu Beispiel 1 wurden aus 1.10 g ( 6.29 mmol) **16** und 2.80 g (6.29 mmol) R,S-2-Decyloxy-3-(5-(4-chlorphenoxy)-pentylmercapto)-1-propanol 1.27 g (36%) R,S-((3-(5-(4-chlorphenoxy)-pentylmercapto)-2-decyloxy)-propoxy)-hydroxyphosphinylameisensäuremethylester (Beispiel 12.31) als farbloses Öl erhalten. Verseifung mit Natronlauge (analog Beispiel 1) ergab 1.34 g (99%) **11** von 7 Konsistenz, Schmp. 175-177°C.

### Beispiel 12

In zu den Beispielen 1 bis 11 analoger Art und Weise können die in Tab. 1 enthaltenen Anwendungsbeispiele 21 bis 51 dargestellt werden.

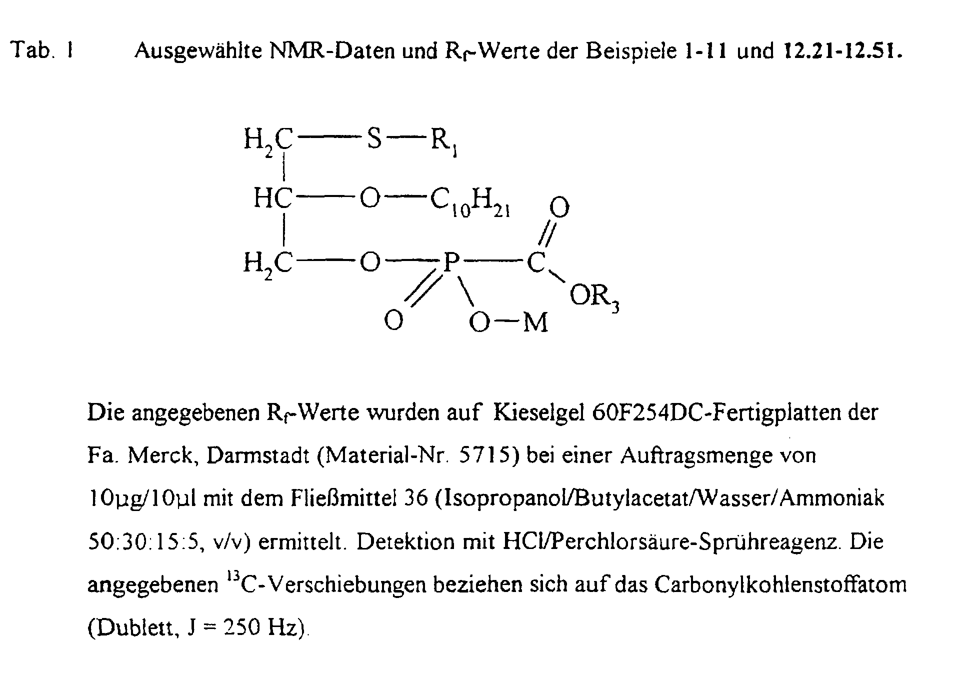

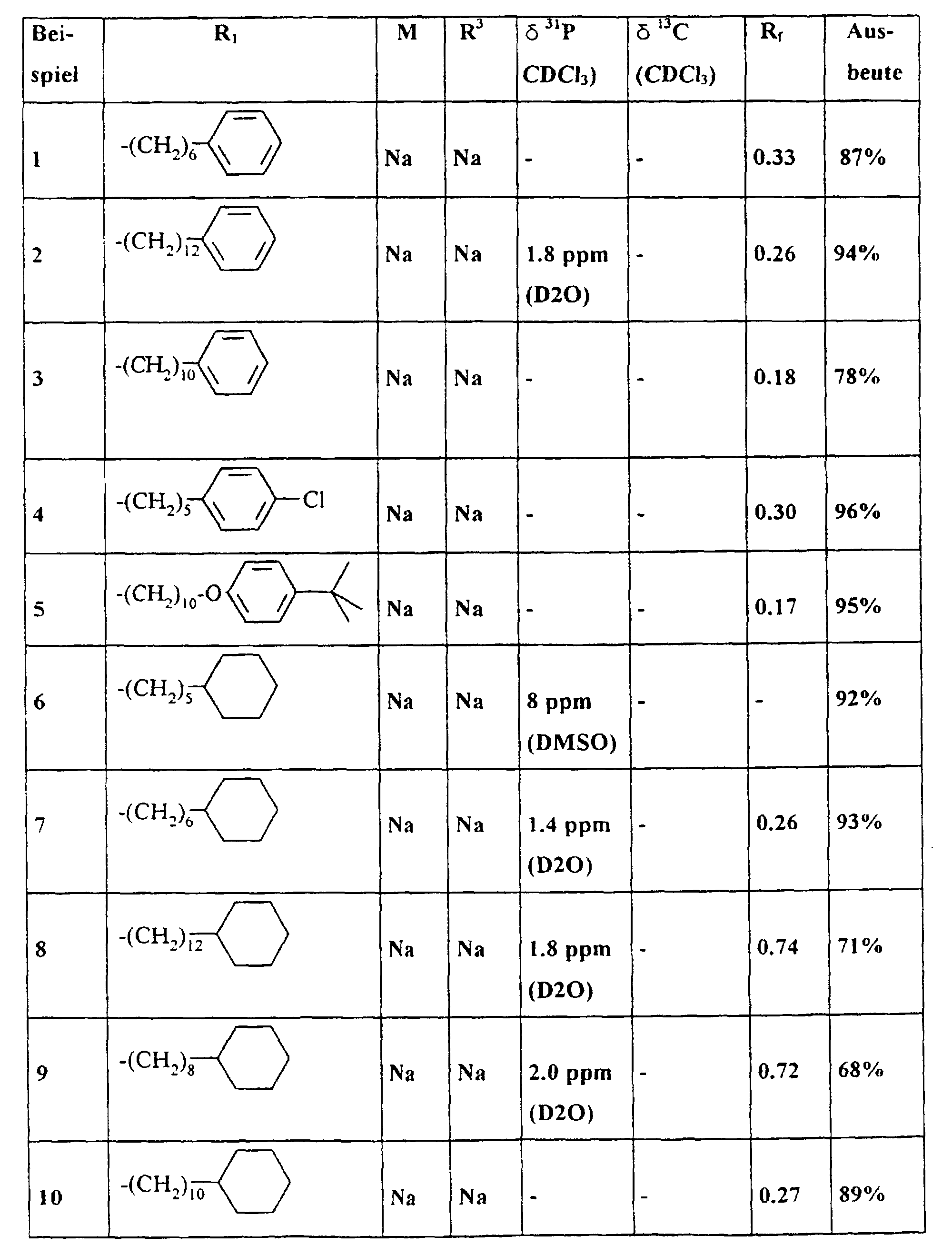

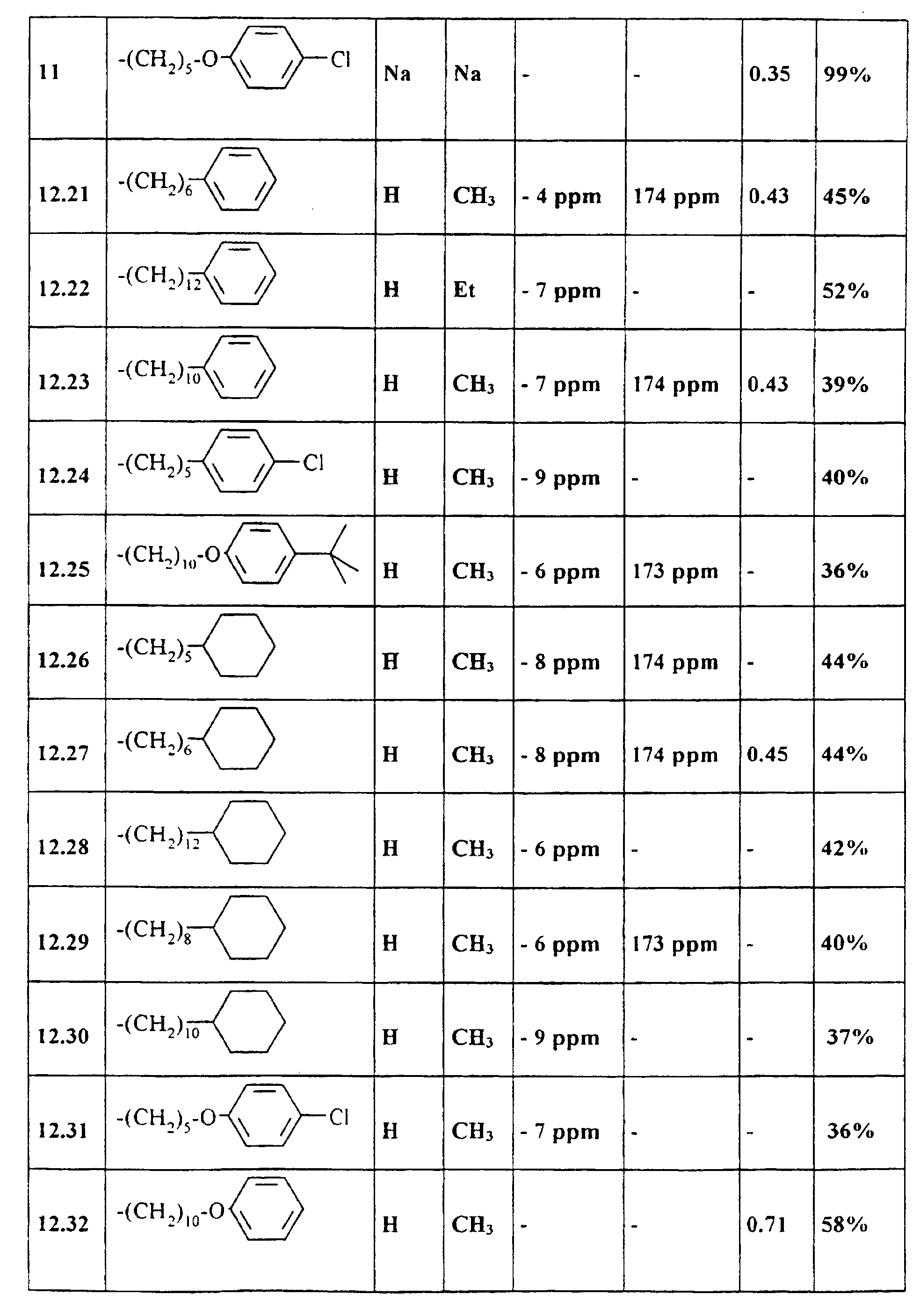

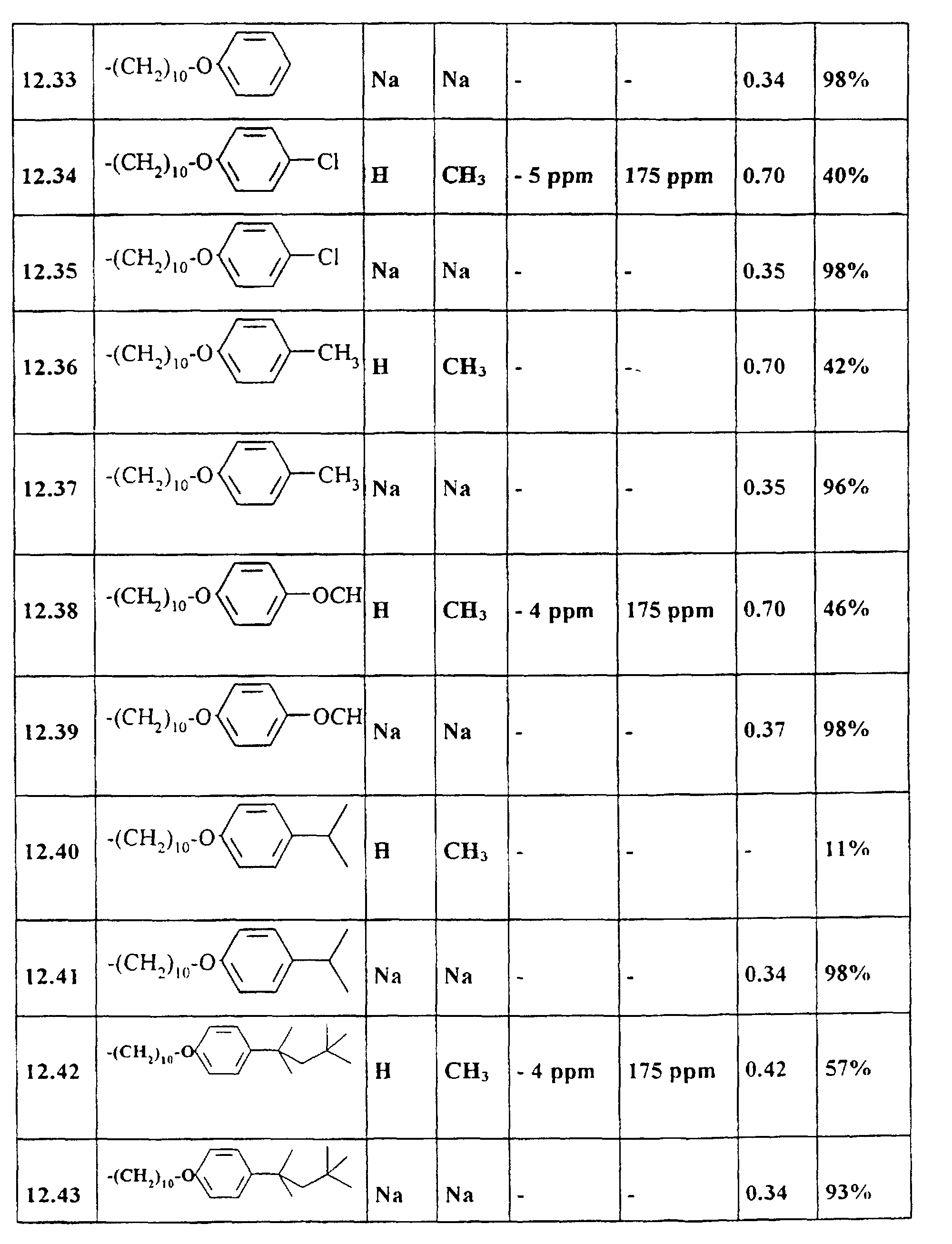

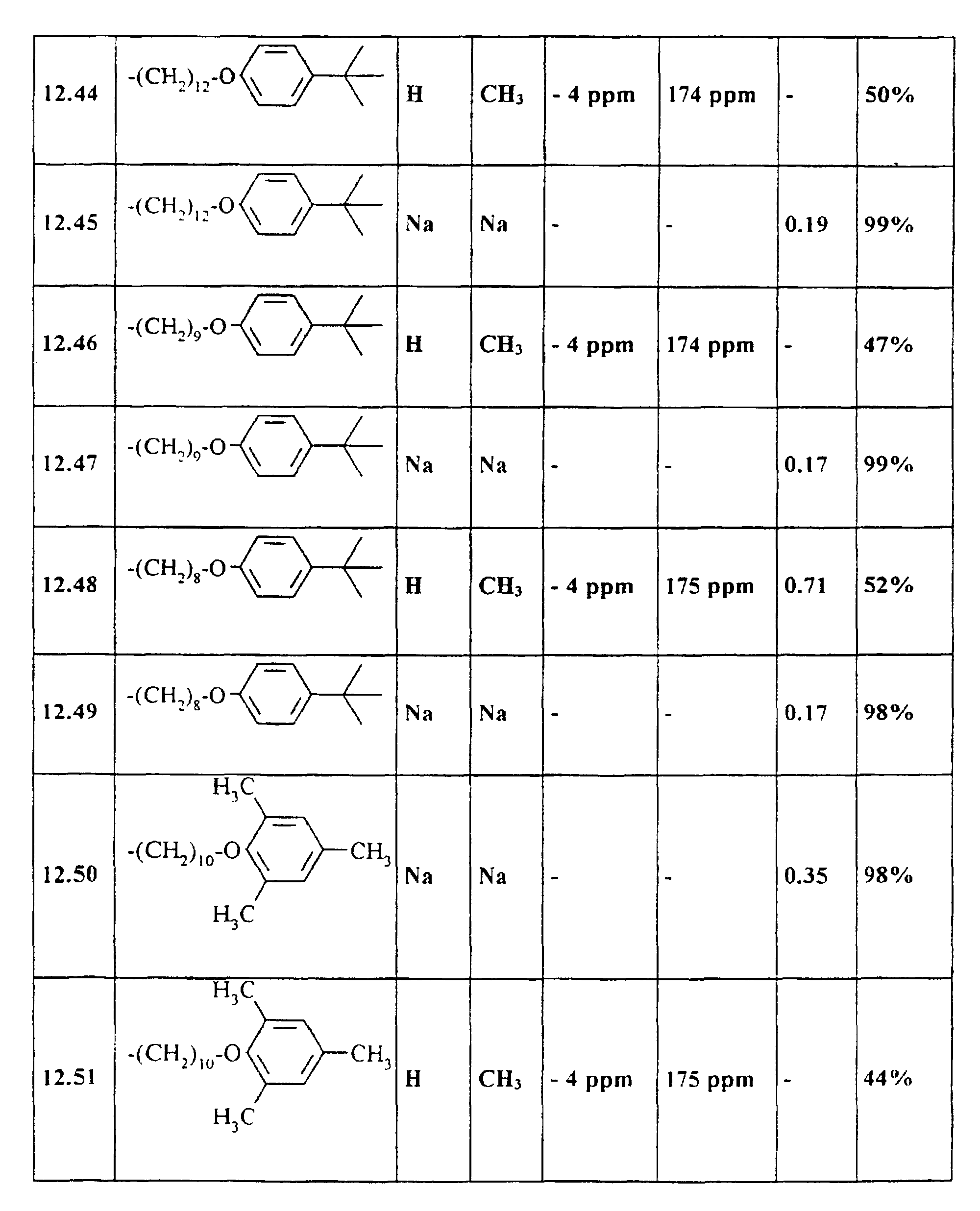

### Beispiel 13

### Testung von Etherlipid-Foscarnet-Konjugaten im murinen Zytomegalievirus (MCMV)-Modell in vivo

Verschiedene Etherlipid-Foscarnet-Konjugate, die Variationen im Etherlipidteil des Moleküls aufweisen, wurden im MCMV-Modell in vivo geprüft. Hierbei wurde i. Vgl. zu scheinbehandelten Kontrollen die Überlebensrate nach Infektion mit MCMV-Virus am Tag +9 nach Infektion bestimmt (Tab. 2).

Die Tiere wurden (außer in Kontrolle I und II) mit 2 x 10⁵ PFUs/Tier i.p. am Tag 0 infiziert. Alle Tiere (außer in Kontrolle I) wurden am Tag -1 mit 100 mg/kg Cyclophosphamid p.o. immunsupprimiert. Alle Testsubstanzen wurden in einer Dosis von 30 mg·kg⁻¹·Tag⁻¹ i.p. täglich einmal von Tag 0 (+1h nach Infektion) bis Tag +8 appliziert. Es wurden jeweils 10 Tiere/Gruppe eingesetzt. Die Zahl der überlebenden Tiere wurde am Tag +9 bestimmt.

Wie aus Tab. 2 hervorgeht, überlebte in der mit PBS scheinbehandelten Kontrolle III (Gruppe 3) lediglich 1 von 10 Tieren bis Tag +9. Alle getesteten Verbindungen waren in diesem Tiermodell wirksam. Hinsichtlich Überlebenszeit ergab sich für die Testsubstanzen eine ausgeprägte Struktur-Wirkungsbeziehung, wobei TBUPHO10S10OP-PFA, CLPH5S10OP-PFA und PH6S10OP-PFA die aktivsten Verbindungen sind.

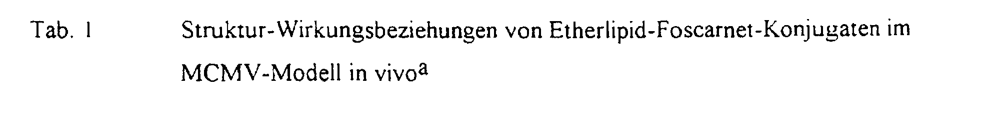

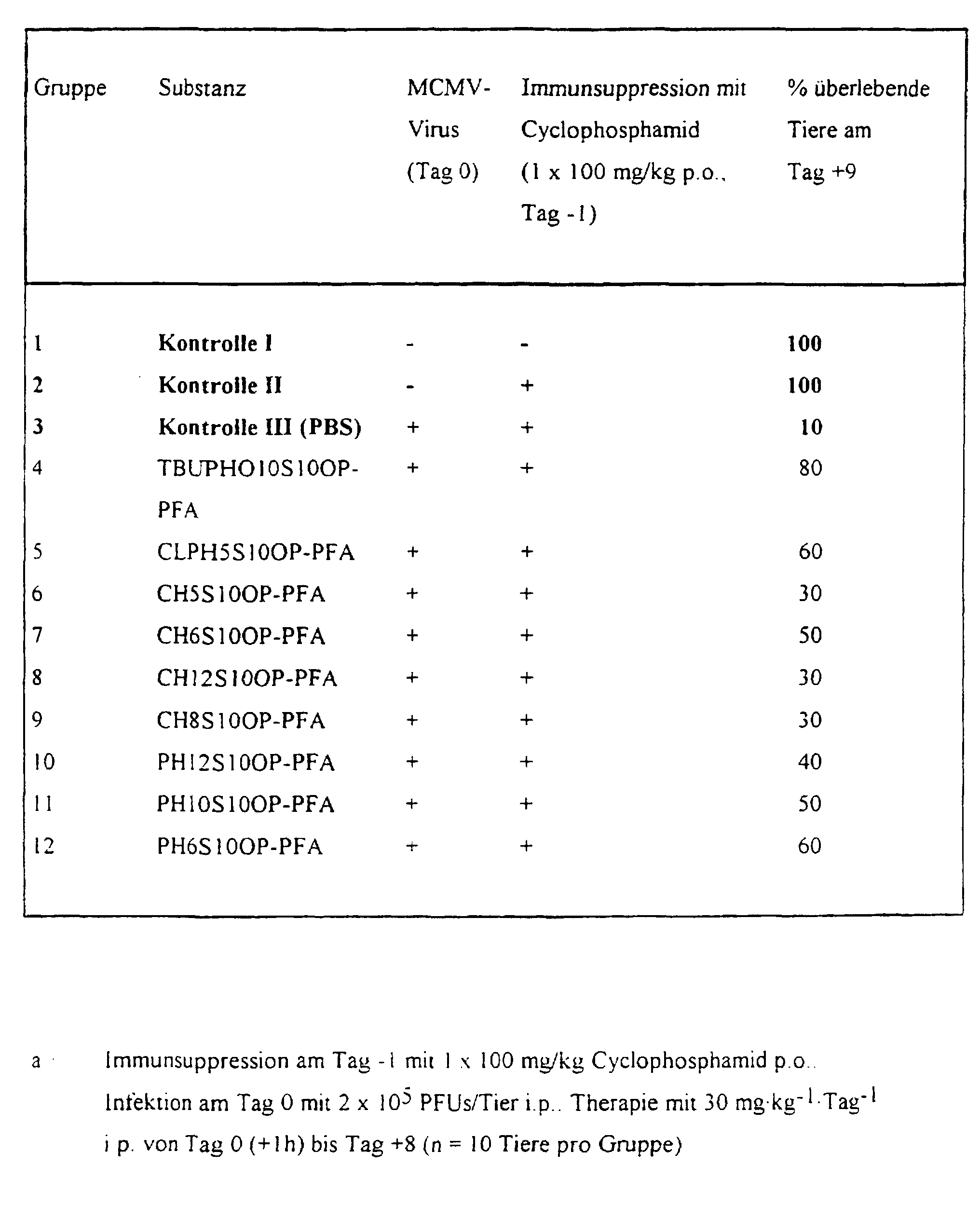

## Patentansprüche

1. Phospholipid-Derivate von Phosphonocarbonsäuren der allgemeinen Formel I, 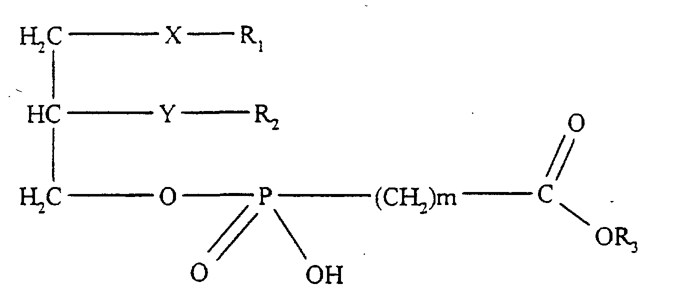 in der
R¹ in einer Gruppierung -(CH₂)ₑ-Cycl eine geradkettige oder verzweigte, gesättigte oder ungesättigte Alkylenkette mit
e gleich einer ganzen Zahl zwischen 4 und 16 entspricht, wobei eines der Kohlenstoffatome ab Position 3 durch ein Heteroatom (Sauerstoff, Stickstoff oder Schwefel) ersetzt sein kann,
R² Wasserstoff oder eine geradkettige oder verzweigte, gesättigte oder ungesättigte Alkylkette mit 1-20 Kohlenstoffatomen sein kann,
R³ H, eine geradkettige oder verzweigte Alkykette mit 1-6 Kohlenstoffatomen, im besondem Methyl, Ethyl, Propyl, Isopropyl, Butyl, Isobutyl, t-Butyl, Pentyl, Hexyl, Neopentyl, Thexyl oder Phenyl, Cholin, Ethanolamin, Carnitin, C₅-C₇-Cycloalkylrest, Benzyl oder eine der folgenden Gruppen 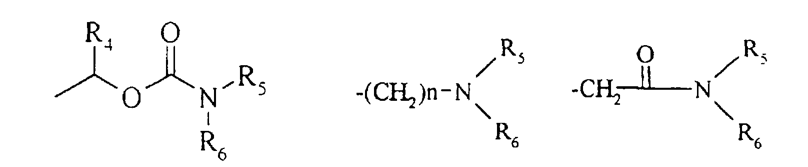 wobei R₄ C₁-C₆-Alkyl, Benzyl oder Phenyl und R₅ und R₆ unabhängig voneinander C₁-C₆-Alkyl und n 1,2 oder 3 bedeuten kann,
X einen Valenzstrich, Sauerstoff, Schwefel, Oxycarbonyl, Carbonyloxy, Carbonylamido, Amidocarbonyl, die Sulfinyl- oder die Sulfonylgruppe bezeichnet,
Y einen Valenzstrich, Sauerstoff, Schwefel, Oxycarbonyl, Carbonyloxy, Carbonylamido, Amidocarbonyl, die Sulfinyl- oder die Sulfonylgruppe bezeichnet,
Cycl einen cyclischen Alkylrest mit 5-7 C-Atomen oder Phenyl darstellt, wobei ein Ring-Kohlenstoffatom durch Stickstoff ersetzt sein kann und die gesättigten oder aromatischen Ringe ein- oder mehrfach durch C₁-C₁₀-Alkyl, C₁-C₁₀-Alkoxy, C₁-C₁₀-Alkylmercapto oder Halogen substituiert sein können,
m 0 bzw für 1 bis 3 steht,
mit der Maßgabe, daß R¹ gleich R² sein kann, wenn R² gleichzeitig die Bedeutung von R¹ annimmt,
deren Tautomere, optischen Isomere, Racemate, deren physiologisch verträgliche Salze anorganischer und organischer Basen und Prodrugs der Verbindungen der allgemeinen Formel I.

2. Verbindungen nach Anspruch 1, in denen R₂ eine geradkettige oder verzweigte, gesättigte oder ungesättigte Alkylkette mit 8-12 Kohlenstoffatomen sein kann.

3. Verbindungen nach einem der Ansprüche 1 oder 2, in denen R₃ nicht Wasserstoff bedeutet.

4. Verbindungen nach einem der Ansprüche 1-3, in denen m für 0, 1 oder 2 steht.

5. Verbindungen nach einem der Ansprüche 1-4, in denen R³ für Methyl, Ethyl, Propyl, Butyl, t-Butyl oder Benzyl steht.

6. Verbindungen nach einem der Ansprüche 1-5, in denen e für 6-10 steht.

7. Verbindungen nach einem der Ansprüche 1-6, in denen X für Schwefel, Sulfinyl, Sulfonyl, Sauerstoff oder einen Valenzstrich steht.

8. Verbindungen nach einem der Ansprüche 1-7, in denen Y für Schwefel, Sulfinyl, Sulfonyl, Sauerstoff oder einen Valenzstrich steht.

9. Verbindung nach einem der Ansprüche 1-8, in denen X gleich Schwefel und Y gleich Sauerstoff bedeutet.

10. Verbindungen nach einem der Ansprüche 1-9, in denen Cycl für Cyclohexyl, Cyclopentyl oder Phenyl steht, das gegebenenfalls durch Halogen oder C₁-C₄-Alkyl substituiert ist.

11. Verbindungen nach einem der Ansprüche 1-10, in denen R² für Nonyl, Dezyl, Undezyl oder Dodecyl steht.

12. Verbindungen nach einem der Anspruche 1-11, in denen R¹ für Phenylhexyl oder Cyclohexylhexyl steht, die gegebenenfalls durch t-Butyl oder Chlor substituiert sind.

13. Arzneimittel enthaltend mindestens eine Verbindung der allgemeinen Formel I nach einem der Ansprüche 1-12 neben üblichen pharmazeutischen Hilfs- und Trägerstoffen.

14. Verwendung mindestens einer Verbindung der allgemeine Formel 1 nach einem der Ansprüche 1-12 zur Herstellung eines Medikaments zur Behandlung von Autoimmunkrankheiten, Neoplasmien, entzündlicher, viraler oder retroviraler Erkrankungen.

## Claims

1. New phospholipid derivatives of phosphonocarboxylic acids of the general formula I, 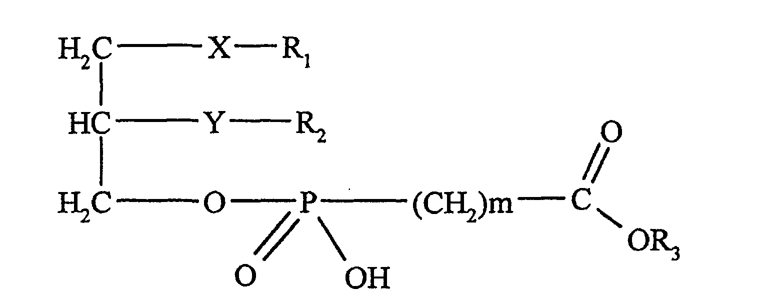 in which
R¹ corresponds to a straight-chained or branched, saturated or unsaturated alkylene chain in a group -(CH₂)ₑ-cycl in which
e is an integer between 4 and 16, and one of the carbon atoms from position 3 onwards can be replaced by a heteroatom (oxygen, nitrogen or sulfur),
R² can be hydrogen or a straight-chained or branched, saturated or unsaturated alkyl chain with 1-20 carbon atoms,
R³ represents H, a straight-chained or branched alkyl chain with 1-6 carbon atoms, preferrably methyl, ethyl, propyl, isopropyl, butyl, isobutyl, t-butyl, pentyl, hexyl, neopentyl, thexyl or phenyl, choline, ethanolamine, camitine, C₅-C₇-cycloalkyl residue, benzyl or one of the following groups 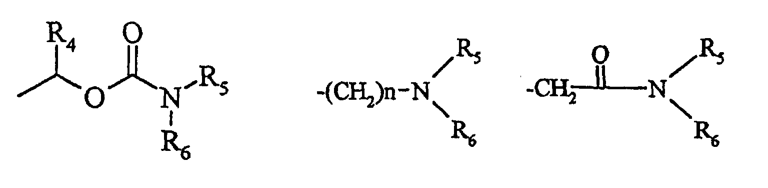 wherein R₄ represents C₁₋C₆-alkyl, benzyl or phenyl and R₅ and R₆ independent of one another C₁-C₆-alkyl and n 1,2 or 3,
X denotes a valency dash, oxygen, sulfur, oxycarbonyl, carbonyloxy, carbonylamido, amidocarbonyl, a sulfinyl or a sulfonyl group,
Y denotes a valency dash, oxygen, sulfur, oxycarbonyl, carbonyloxy, carbonylamido, amidocarbonyl, a sulfinyl or sulfonyl group,
Cycl represents a cyclic alkyl residue with 5-7 C atoms or phenyl in which one ring carbon atom can be replaced by nitrogen and the saturated or aromatic rings can be substituted once or several times by C₁-C₁₀ alkyl, C₁-C₁₀ alkoxy, C₁-C₁₀ alkylmercapto or halogen,
m represents 0, 1, 2, or 3,
provided that R¹ can be the same as R² if R² at the same time has the meaning of R¹,
tautomers, optical isomers and racemates thereof and their physiologically tolerated salts of inorganic and organic bases and prodrugs of compounds of the formula I.

2. Compound of claim 1 in which R₂ represents a straight-chained or branched, saturated or unsaturated alkyl chain with 8 to 12 carbon atoms..

3. Compound of one of the clains 1 or 2 in which R₃ is not hydrogen.

4. Compound of one of the claims 1 to 3 in which m represents 0, 1 or 2.

5. Compound of one of the claims 1-4 in which R₃ represents methyl, ethyl, propyl, butyl, t-butyl or benzyl.

6. Compound of one of the claims 1-5 in which e respresents a number between 6 and 10.

7. Compounds of one of the claims 1-6 in which X represents sulfur, sulfinyl, sulfonyl, oxygen or a valence dash.

8. Compounds of one of the claims 1-7 in which Y represents sulfur, sulfinyl, sulfonyl, oxygen or a valence dash.

9. Compound of one of the claims 1 to 8 in which X represents sulfur and Y represents oxygen.

10. Compound of one of the claims 1 to 9 in which cycl represents cyclohexyl, cyclopentyl or phenyl which is optionally substituted by halogen or C₁-C₄ alkyl.

11. Compound of one of the claims 1-10 in which R² represents nonyl, decyl, undecyl or dodecyl.

12. Compounds of one of the claims 1-16 in which R1 represents phenylhexyl or cyclohexylhexyl which is optionally substituted by t-butyl or chlorine.

13. Pharmaceutical composition containing at least one compound of the general formula I as claimed in one of the claims 1 to 12 in addition to the usual pharmaceutical auxiliary substances and carriers.

14. Use of at least one compound of the general formula I as claimed in one of the claims 1 to 12 for the production of a medicine for the treatment of autoimmune diseases, neoplasias, inflammatory, viral or retroviral diseases.

## Revendications

1. Dérivés phospholipides d'acides phosphonocarboxyliques de formule générale I, 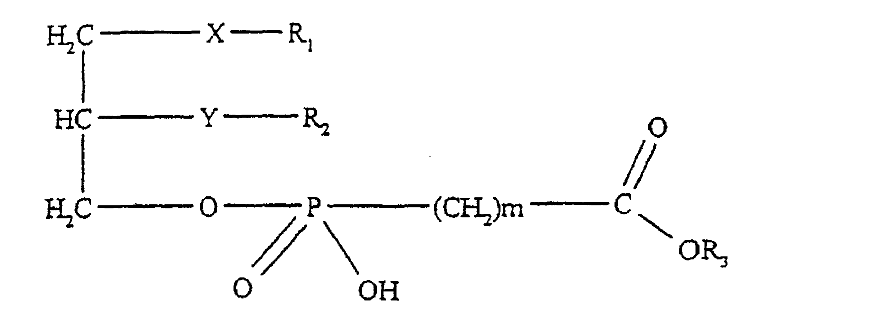 dans laquelle
R¹ dans un groupement -(CH₂)ₑ-Cycl, correspond à une chaîne alkylène saturée ou insaturée, à chaîne droite ou ramifiée,
e étant égal à un nombre entier compris entre 4 et 16, où un des atomes de carbone à partir de la position 3 peut être remplacé par un hétéroatome (atome d'oxygène, d'azote ou de soufre),
R² peut être un atome d'hydrogène ou une chaîne alkyle saturée ou insaturée, à chaîne droite ou ramifiée ayant 1 à 20 atomes de carbone,
R³ peut représenter un atome de H, une chaîne alkyle à chaîne droite ou ramifiée ayant 1 à 6 atomes de carbone, en particulier un groupe méthyle, éthyle, propyle, isopropyle, butyle, isobutyle, t-butyle, pentyle, hexyle, néopentyle, thexyle ou phényle, choline, éthanolamine, carnitine, résidu cycloalkyle en C₅ à C₇, benzyle ou un des groupes suivants 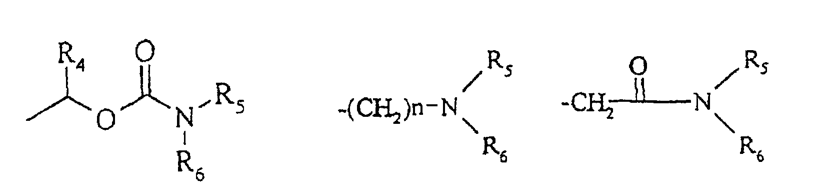 dans lesquels R⁴ peut représenter un groupe alkyle en C₁ à C₆, benzyle ou phényle, et R⁵ et R⁶ indépendamment l'un de l'autre peuvent représenter un groupe alkyle en C₁ à C₆, et n vaut 1, 2 ou 3,
X représente une liaison de valence, un atome d'oxygène, de soufre, un groupe oxycarbonyle, carbonyloxy, carbonylamido, amidocarbonyle, le groupe sulfinyle ou le groupe sulfonyle,
Y représente une liaison de valence, un atome d'oxygène, un atome de soufre, un groupe oxycarbonyle, carbonyloxy, carbonylamido, amidocarbonyle, le groupe sulfinyle ou le groupe sulfonyle,
Cycl représente un résidu alkyle cyclique ayant 5 à 7 atomes de C ou un groupe phényle, dans lequel un atome de carbone du cycle peut être remplacé par un atome d'azote et les cycles saturés ou aromatiques peuvent être substitués une ou plusieurs fois par un groupe alkyle en C₁ à C₁₀, alcoxy en C₁ à C₁₀, alkylmercapto en C₁ à C₁₀, ou halogéno,
m vaut 0 ou représente un nombre de 1 à 3,
avec la condition que R¹ puisse être égal à R², lorsque R² prend simultanément la signification de R¹,
leurs tautomères, isomères optiques, racémates, leurs sels avec des bases inorganiques et organiques, acceptables sur le plan physiologique et les promédicaments des composés de formule générale I.

2. Composés selon la revendication 1, dans lesquels R² peut être une chaîne alkyle saturée ou insaturée, à chaîne droite ou ramifiée avec 8 à 12 atomes de carbone.

3. Composés selon l'une quelconque des revendications 1 ou 2, dans lesquels R³ ne représente pas un atome d'hydrogène.

4. Composés selon l'une quelconque des revendications 1 à 3, dans lesquels m représente 0, 1 ou 2.

5. Composés selon l'une quelconque des revendications 1 à 4, dans lesquels R³ représente un groupe méthyle, éthyle, propyle, butyle, t-butyle ou benzyle.

6. Composés selon l'une quelconque des revendications 1 à 5, dans lesquels e vaut de 6 à 10.

7. Composés selon l'une quelconque des revendications 1 à 6, dans lesquels X représente un atome de soufre, un groupe sulfinyle, sulfonyle, un atome d'oxygène ou une liaison de valence.

8. Composés selon l'une quelconque des revendications 1 à 7, dans lesquels Y représente un atome de soufre, un groupe sulfinyle, sulfonyle, un atome d'oxygène ou une liaison de valence.

9. Composé selon l'une quelconque des revendications 1 à 8, dans lesquels X est identique à un atome de soufre et Y est identique à un atome d'oxygène.

10. Composés selon l'une quelconque des revendications 1 à 9, dans lesquels Cycl représente un groupe cyclohexyle, cyclopentyle ou phényle, qui est éventuellement substitué par un atome d'halogène ou un groupe alkyle en C₁ à C₄.

11. Composés selon l'une quelconque des revendications 1 à 10, dans lesquels R² représente un groupe nonyle, décyle, undécyle ou dodécyle.

12. Composés selon l'une quelconque des revendications 1 à 11, dans lesquels R¹ représente un groupe phénylhexyle ou cyclohexylhexyle, qui sont éventuellement substitués par un groupe t-butyle ou un atome de chlore.

13. Médicament contenant au moins un composé de formule générale 1 selon l'une quelconque des revendications 1 à 12 en plus des adjuvants et véhicules pharmaceutiques habituels.

14. Utilisation d'au moins un composé de formule générale I selon l'une quelconque des revendications 1 à 12, pour préparer un médicament en vue de traiter les maladies auto-immunes, les néoplasies, les maladies inflammatoires, virales ou rétrovirales.
